# EUROPEAN PATENT APPLICATION

(11) **EP 4 197 437 A1**
(43) Date of publication of application: **21.06.2023**
(21) Application number: 21215680.6
(22) Date of filing: 17.12.2021
(51) Int. Cl.: A61B 5/07, A61B 5/00, A61B 5/145

(54) **METHODS AND DEVICES FOR IN VIVO ASSESSMENT OF ANALYTES IN THE GASTROINTESTINAL TRACT**

(71) Applicant: Stichting IMEC Nederland, 6708 WH Wageningen (NL)
(72) Inventor: EVEN, Aniek, 6811 LW Arnhem (NL); LEONARDI, Francesca, 3901 RB Veenendaal (NL); VAN HELLEPUTTE, Nick, 3060 Korbeek-Dijle (BE); ARMSTRONG, Rachel, 5616 HB Eindhoven (NL)
(74) Representative: AWA Sweden AB

(57) **Abstract**

The current disclosure relates to methods and devices for measuring concentrations of analytes in the gastrointestinal tract of a subject by orally administering a smart pill to the subject, the smart pill comprising two or more sensors, a reference electrode, a power source, a communication interface and electronic circuits, each sensor being able to measure an analyte in the gastrointestinal tract of said subject, wherein the two or more sensors measure different analytes, measuring a concentration of two or more analytes using the two or more sensors, wherein one of the sensors is a pH sensor for measuring hydrogen ions, and wherein the pH sensor is able to locate the smart pill in the gastrointestinal tract by correlating the measured hydrogen ion concentration to a location in the gastrointestinal tract, and transmitting, using the communication interface, the measured concentrations from the two or more sensors to a base device located outside of the body of the subject.

## Description

### TECHNICAL FIELD

The present disclosure relates to methods and devices for measuring concentrations of analytes in the gastrointestinal tract of a subject. More specifically, the proposed technique relates to smart pills comprising ingestible sensors for measuring analytes and assessing gut health *in vivo.* Methods for measuring analytes and assessing gut health, and smart pills for enabling said methods, are disclosed.

### BACKGROUND

The human gastrointestinal (GI) system is a complex and difficult to access environment. Current research methods for measuring analytes and assessing gut health are either invasive (e.g. endoscopy), require expensive equipment, give only limited information (e.g. fecal analysis) or lack sensitivity. As gut health can have crucial impact on people's general health and is linked to a number of conditions and disorders, enhanced methods for measuring analytes to be able to assess gut health are needed.

### SUMMARY

An object of the present disclosure is to provide methods and devices which seek to mitigate, alleviate, or eliminate the above-identified deficiencies in the art and disadvantages singly or in any combination. This object is obtained by a smart pill and a method for measuring concentrations of analytes in a gastrointestinal tract of a subject, the method comprising orally administering a smart pill to the subject, the smart pill comprising two or more sensors, a reference electrode, a power source, a communication interface and electronic circuits, each sensor being able to measure a concentration of an analyte in the gastrointestinal tract of the subject, wherein the two or more sensors measure different analytes, measuring a concentration of two or more analytes using the two or more sensors, wherein one of the sensors is a pH sensor for measuring hydrogen ion concentrations as a concentration of one analyte, and wherein the pH sensor is able to locate the smart pill in the gastrointestinal tract by correlating the measured hydrogen ion concentrations to a location in the gastrointestinal tract, and transmitting, using the communication interface, the measured concentrations from the two or more sensors to a base device located outside of a body of the subject.

According to some aspects, the method further comprises transmitting the measured concentrations from the base device to a processing device or a cloud infrastructure, and assessing gut health of the subject based on the measured concentrations of analytes of the two or more sensors using the processing device or cloud.

According to some aspects, the method may further comprise calibrating the two or more sensors before oral administration to the subject.

According to some aspects, the disclosure proposes a smart pill configured to operate inside a gastrointestinal tract of a subject and being capable of measuring analyte concentrations therein, the smart pill comprising a power source, two or more sensors being configured to measure concentrations of different analytes inside the gastrointestinal tract of the subject, where at least one of the two or more sensors comprises a non-degradable membrane, the smart oil further comprising a reference electrode, a communication interface comprising a transceiver for enabling wireless communication between the smart pill and a base device, and being configured to transmit and receive data between the sensors and the base device, electronic circuits for enabling a connection between the sensors, the reference electrode and the communication interface, and being configured to convey data from the sensors to the communication interface.

In some aspects, the smart pill is configured to perform the methods of measuring concentrations of analytes and assessing gut health as described above. In some aspects, the two or more sensors of the smart pill are selected from a pH sensor, an ammonium sensor and a nitric oxide sensor for measuring analytes such as hydrogen ions, ammonium ions and nitric oxide. In some aspects, one or more additional sensors are included, which may be able to correct for interference of other compounds than the analytes measured. A sub-group of said other compounds may be background ions. An example of such background ions are potassium and sodium ions, where potassium ions would be useful to measure especially for the ammonium sensor. For the nitric oxide sensor, examples of interfering compounds that could be measured are dopamine, ascorbic acid, and nitrites.

Other objects and advantages will become apparent to those skilled in the art from a review of the ensuing detailed description, which proceeds with reference to the following illustrative drawings, and the attendant claims.

Generally, all terms used in the claims are to be interpreted according to their ordinary meaning in the technical field, unless explicitly defined otherwise herein. All references to "a/an/the" element, device, component, step, etc. are to be interpreted openly as referring to at least one instance of said element, device, component, step, etc., unless explicitly stated otherwise. The steps of any method disclosed herein do not have to be performed in the exact order disclosed, unless explicitly stated.

### BRIEF DESCRIPTION OF THE DRAWINGS

The above, as well as additional objects, features and advantages of the present inventive concept, will be better understood through the following illustrative and non-limiting detailed description of different embodiments of the present inventive concept, with reference to the appended drawings.

The figures are not necessarily to scale, and generally only show parts that are necessary in order to elucidate the inventive concept, wherein other parts may be omitted or merely suggested.
Figure 1 shows a flow chart of a method of using a smart pill comprising sensors (ingestible sensor) for measuring pH and ammonium ions to assess the presence of an H. pylori infection.
Figure 2 is an illustration of a set-up for a study using a smart pill (ingestible sensor) for measuring pH and ammonium ions.
Figure 3 depicts a smart pill of the invention according to different embodiments. A smart pill comprising two sensors, pH sensor and ammonium sensor, is depicted in figure 3A, and a smart pill comprising three sensors, pH sensor, ammonium sensor and nitric oxide, is depicted in figure 3B.
Figure 4 shows an illustration of sensors of the present disclosure, where 4A depicts a pH sensor using ISFET and 4B depicts an ammonium sensor using ChemFET.
Figure 5 illustrates an ingestible sensor (smart pill) comprising a pH sensor and an ammonium sensor showing the membrane deposition.
Figure 6A shows an example of a working mechanism for the ammonium sensor using an ISFET, and Figure 6B illustrates the relationship of concentration to measured potential of the ammonium sensor. Figure 6C illustrates the relationship of concentration to measured potential of the pH sensor.
Figure 7 shows an example of the ammonium ion sensor being an ion selective electrode (ISE).
Figure 8 illustrates the selectivity, 8A, and measured signal at two nitric oxide levels, 8B, of a nitric oxide sensor of the present disclosure.
Figure 9 is a schematic drawing of the different components of the smart pill system.
Figure 10 illustrates the system comprising the smart pill inside the body of a patient, and the base device outside of the body of the patient.
Figure 11 is a flow chart of a method of measuring analytes and assessing gut health according to an aspect of the present disclosure.
Figure 12 is a block diagram of a smart pill of the present disclosure.
Figure 13 is a schematic overview of an embodiment of the invention, where pH, ammonium ions and nitric oxide are being measured in patients suspected of having a Helicobacter pylori infection.
Figure 14 is a schematic overview of an embodiment of the invention, where pH and nitric oxide are being measured in a patient having inflammatory bowel disease.
Figure 15 is a schematic overview of an embodiment of the invention, where pH and ammonium ions are being measured in a patient to monitor protein fermentation.

### DETAILED DESCRIPTION

Aspects of the present disclosure will be described more fully hereinafter with reference to the accompanying drawings. The apparatus and method disclosed herein can, however, be realized in many different forms and should not be construed as being limited to the aspects set forth herein. Like numbers in the drawings refer to like elements throughout.

The terminology used herein is for the purpose of describing particular aspects of the disclosure only, and is not intended to limit the disclosure. As used herein, the singular forms "a", "an" and "the" are intended to include the plural forms as well, unless the context clearly indicates otherwise.

In some embodiments a non-limiting term "smart pill" is used. The term "ingestible sensor" or "ingestible device" may also be used interchangeably. The smart pill or ingestible sensor/device of the present disclosure relates to a small device or entity which comprises two or more sensors, and which may be ingested by a subject, such as swallowed by a patient in need thereof. The smart pill comprises a wireless communication interface, comprising a transceiver (transmitter and receiver) that may transmit information from the sensors to a receiver outside of the body of the subject which has ingested the pill. The smart pill will also typically comprise some sort of powering/energy source, such as batteries, electronic circuits or processing circuitry, such as electronic read-out circuits, and a reference electrode used as a reference for the sensors.

The term "sensor" as used herein refers to an entity that detects and measures analytes. The sensors of the present disclosure may include a "non-degradable membrane", which means that the membrane will not be degraded upon contact with the analyte, and hence may be used for repeated and continuous measurements, as compared to a degradable membrane which are used once and then needs to be replaced. The membrane may be made of a polymeric material which is non degrading in the environment, where the polymeric material is resistant to the environmental conditions (pH, temperature, pressure, etc.) which it is being exposed to in the gastrointestinal tract. The membrane may be said to be selective or sensitive to a specific analyte, which means that it allows measurements of said analyte (indirectly by measuring the potential), which may be correlated to a concentration of the analyte. The sensor may include ion selective field effect transistors (ISFET) combined with a reference electrode, an ammonium selective field effect transistor (ChemFET) or a sensor being an ion selective electrode (ISE), etc. In some aspects, the sensors measure pH, ammonium ions and nitric oxide, respectively.

The term "analytes" as used herein refers to a substance or chemical constituent that is of interest for further analysis. The analytes as measured by the sensors of the smart pill may be assessed and linked to different aspects or conditions of the gastrointestinal tract. The analytes may include pH, ammonium ions, nitric oxide and potassium ions, etc.

The "concentrations" of the analytes, their level of occurrence in a certain sample, are measured by the sensors. The concentrations may be measured indirectly, by measuring for example a raw potential or current in the sensor, which may be correlated to a concentration. In this case the raw data signal (potential/current) is transmitted from the sensors to the base device.

The term "gut health" as used herein refers to a state or condition in the gastrointestinal tract which affects the wellbeing of the subject. These conditions may include infection with different bacteria, such as Helicobacter pylori (also referred to as H. pylori interchangeably), the presence of ulcers or inflammation, and increased occurrence of protein fermentation, etc., which may be linked to disorders such as inflammatory bowel disease, Ulcerative Colitis and Crohn's Disease. The "subject" as referred to herein is typically a human, such as a healthcare patient, but may also be a non-human mammal, such as a veterinary patient.

The term "threshold" or "predetermined threshold" as used herein refers to a value at or above which a concentration of an analyte will be considered as being significantly elevated, which may be indicative of the presence of a state or condition affecting gut health. In some cases, a "reference value" of an analyte is used instead, which may be predetermined based on general knowledge, or which may be calibrated for a specific subject based on earlier measurements of said analyte in said subject, i.e. calibrated in comparison to measurements of said analyte in a specific subject. Thus, a "significant elevation" of an analyte may either be in comparison to a threshold or reference value, or may be based on baseline measurements of an analyte.

The gastrointestinal (GI) tract, also referred to as the gut, is a pathway by which food enters the body and solid wastes are expelled, and includes the mouth, pharynx, esophagus, stomach, small intestine, large intestine, and anus. A healthy gut contains healthy bacteria and immune cells that ward off infectious agents like bacteria, viruses and fungi. A healthy gut also communicates with the brain through nerves and hormones, which helps maintain general health and well-being. The human gut microbiome, the collection of microorganisms residing in the gastrointestinal tract, is thought to play a role in the etiology of various diseases, including inflammatory bowel disease, type 2 diabetes, hypertension, and colorectal cancer. Gut health has even been linked to anxiety and depression, and to neurological conditions like schizophrenia and dementia.

Changes in gut microbiota have been linked with various metabolic and inflammatory diseases and cancers. One common cause of these conditions are caused by an infection with Helicobacter pylori (H. pylori), which is a bacterium that can be found in the human stomach. An infection with H. pylori can lead to stomach inflammation (gastritis), ulcers of the stomach or first part of the small intestine, and some types of cancers. H. pylori might play an important role in the stomach ecosystem and has been associated with a wide range of diseases, not limited to the stomach. Current methods for detecting H. pylori infection include invasive methods, where gastric biopsy samples are taken during an endoscopy, and non-invasive breath tests with labelled 13C- or 14C-urea, or stool analysis. Thus, the problems with current H. pylori detection methods are that they are either invasive (require endoscopic procedure), or for the non-invasive methods, they require labeled material and expensive equipment (13C- or 14C-urea breath test), or have low sensitivity (stool antigen tests). Thus, enhanced methods for assessing H. pylori infection are needed.

Another condition which is linked to inflammation of the GI-tract is inflammatory bowel disease (IBD). Inflammatory bowel disease is the overarching term for Crohn's Disease (CD) and Ulcerative Colitis (UC). Both diseases are characterized by inflammation along the GI tract. In CD inflammations can be anywhere along the gut; in UC the inflammations are mainly located in the proximal part of the colon. The location and extent of the inflammation can be difficult to assess. Clinical evaluation is now mainly relying on invasive endoscopic examinations, fecal analysis, or evaluation of symptoms.

IBD patients often suffer from nutritional deficiencies because the uptake of nutrients is reduced, and patients might avoid some food groups to prevent new flare-ups. After a disease episode extra protein could help heal the mucosa, however, excess intake of protein can lead to high levels of protein fermentation in the colon resulting in detrimental effects and potentially fuel the inflammation. High, or increased levels of, protein fermentation (ammonium is an indication of protein fermentation) has been associated with development of UC. Thus, methods for assessing inflammation status and simultaneous finetuning of nutritional intake would be very beneficial for these patients.

To address the drawbacks of the previous methods for measuring analytes and assessing gut health, a method of using ingestible sensors in a smart pill by embedding the sensors within the pill to measure analytes and assess gut health *in vivo* is proposed.

This method is non-obstructive, and since the ingestible sensors of the smart pill will travel through the gut via the natural route, it can collect data from the whole GI tract, and data can be live transmitted from the smart pill to a receiver outside the body. A smart pill comprising multiple sensors will be able to both locate the smart pill in the GI-tract, and also measure one or more markers for gut health.

Thus in one embodiment is proposed a smart pill comprising at least two sensors, where one of the sensors is a pH sensor. The at least two sensors, including the pH sensor, may be integrated in a smart pill together with a reference electrode, which has a stable and well-known electrode potential. The reference electrode may be an aqueous reference electrode, such as an Ag/AgCI reference electrode (silver chloride electrode). pH may be measured for example with a potentiometric technique based on ion selective field effect transistors (ISFET) combined with the reference electrode. The pH measurements/sensing will be used for determining the location of the smart pill within the GI-tract, but may also serve as a general indicator of gut health. The pH of the stomach may for example be indicative of gut heath, where a too acidic stomach may be treated using proton pump inhibitors to reduce the acidity of the stomach. These may be patients with for example peptic ulcers, gastroesophageal reflux disease, gastritis, and H pylori. The proton pump inhibitor drugs might have side effects though, influencing digestion, uptake of vital vitamins/minerals, etc. Thus, being able to treat the cause behind the acidity would be beneficial. Also, pH is indicative of the production of short-chain fatty acids (SCFA). SCFA are the main product of gut microbiota and in general associated with improved (gut) health.

The method of measuring analytes and assessing gut health in a patient or subject in need thereof may start with the patient swallowing the smart pill including the sensors. Within seconds after swallowing, it will end up in the stomach and will normally stay in the stomach for roughly 2 hours. Positioning in the stomach can be confirmed with the pH sensor, as the pH in the stomach is very different from the rest of the GI tract, being approximately pH 2 in the stomach, as compared to about pH 6 in the duodenum, for example.

In one aspect, a smart pill comprising at least two sensors may be used for assessing H. pylori infection. In one embodiment, two sensors are used, one for assessing pH as described above, and one for detecting ammonium ions (NH₄⁺). Ammonium is an end product of multiple bacterial processes, such as the conversion of proteins or urea. Increased protein fermentation is associated with IBD, and protein fermentation products are associated with impaired gut health. H. pylori produces the enzyme urease, which can convert urea into ammonium ions. Thus, conversion of urea into ammonium in the stomach is a sign of H. pylori activity, i.e. H. pylori infection. The effect of the conversion of urea into ammonium by H. pylori in the stomach was shown in a publication by Chittajallu et al. Gut, 32(1), 21-24 (1991). Gastric ammonium concentration was compared between patients with an active H. pylori infection to patients after eradication of the infection. Urea was administered to both groups and their gastric juice was sampled every 15 min. Distinctly different ammonium profiles can be observed between the patients with the H. pylori infection and without the infection. The procedure was performed using an invasive endoscopic procedure, which will be avoided using the smart pill method. The principle that H. pylori converts urea into ammonium is used in the prior art to detect H. pylori using breath tests, or on a sample that has been taken during endoscopic procedures. With the methods of the present disclosure it is possible to directly measure it in the stomach. Accordingly, in the present disclosure ammonium ions levels and pH may be measured with a potentiometric technique based on ion selective field effect transistors (ISFET) using sensors integrated in a smart pill. The ammonium sensor and pH sensor may be integrated in a smart pill together with a reference electrode, as described above.

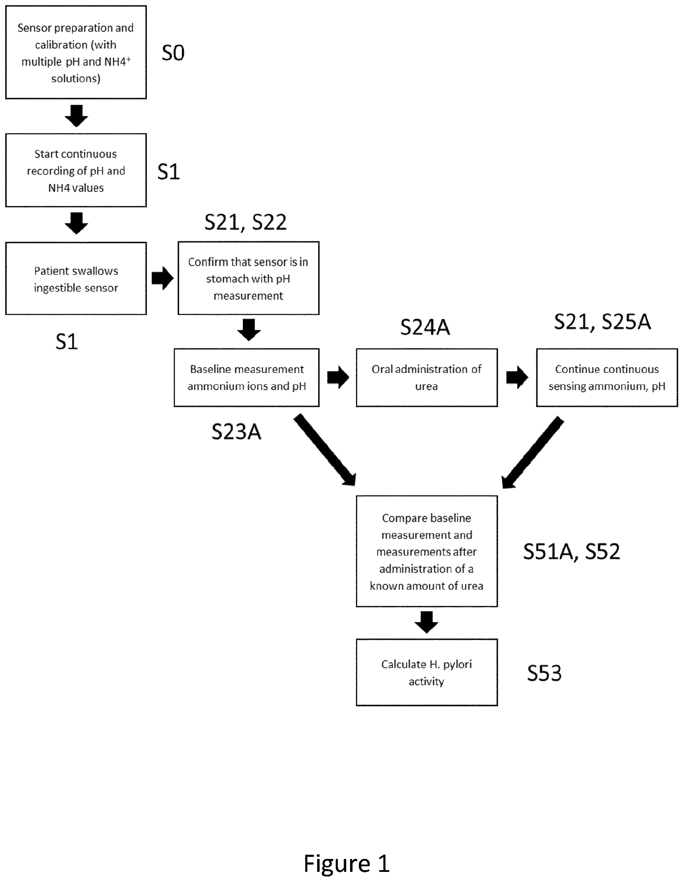

To detect the presence of H. pylori, measurements before and after urea administration may be measured. Baseline stomach measurements of ammonium ions and pH will be recorded. A known concentration of urea will be administered orally. Measurements after administration of urea will be compared with the baseline measurements to derive the urease activity and thereby H. pylori. The algorithm used in the invention can calculate the H. pylori activity based on the baseline values and known concentration of urea, as illustrated in Figure 1. The ammonium concentration will remain stable if no H. pylori is present and will increase in the presence of the infection.

Thus, this embodiment includes a potentiometric readout for the monitoring of pH and ammonium ions. It includes a reference electrode, such as a silver/silver chloride reference electrode. It could be equipped with calibration and temperature compensation systems. The potentiometric readout circuit for monitoring pH along the GI tract may utilize field effect transistor (FET)-based sensors in the form of ion-sensitive field effect transistor (ISFETs). The potentiometric readout circuit for the monitoring of ammonium ions along the GI tract may utilize ammonium-selective FET-based sensors in the form of ISFETs or ammonium-selective ion selective electrodes (ISE). The ammonium-selective ISFET sensor is referred to as ChemFET.

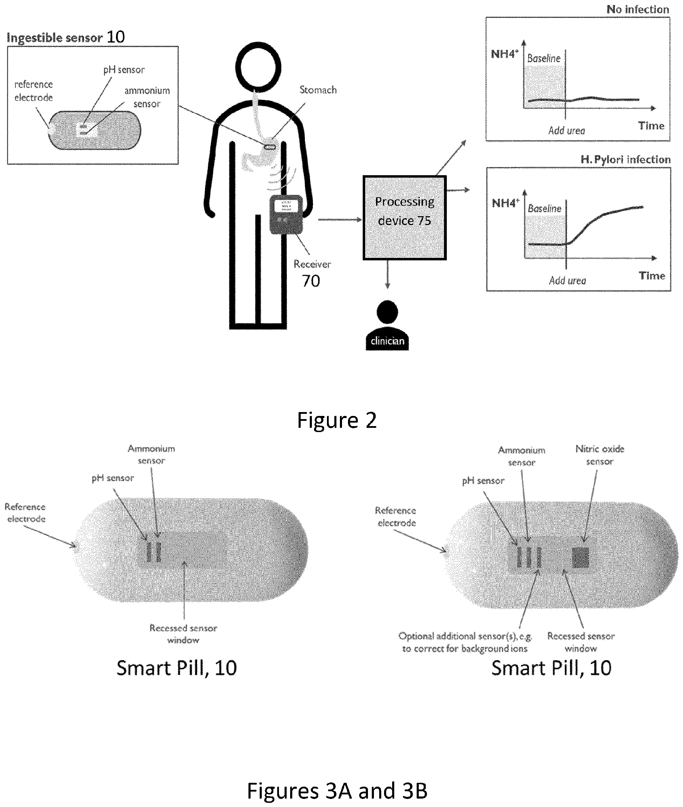

Figure 2 depicts a set-up for using a smart pill (ingestible sensor) for assessing H. pylori activity by measuring pH and ammonium ions, the patient has taken the smart pill/ingestible sensor, which is now in the stomach. Data will be recorded continuously for the whole duration of the pill in the gastrointestinal tract. The smart pill will transmit the pH and ammonium data wirelessly to a receiver base station outside the body. The receiver base station may include a base device 70 and a processing device 75. In one embodiment, the processing device 75 may be communicatively coupled to the base device 70, e.g. via the cloud. In another embodiment, the processing device 75 may be included in the base device 70 or part of the base device 70. Data will be transmitted throughout the whole gut transit and recorded on the base device. The smart pill will stay in the stomach for roughly 2 hours. The data can be viewed by the clinician. The H. pylori activity can be derived from the ammonium graphs over time.

A drawback of measuring ammonium ions only for assessing H. Pylori activity in the prior art is that some patients may be false positives, and many people have the infection, but will never get symptoms. To prevent overtreatment, patient stratification is important. Thus, in another embodiment, three sensors are integrated into the smart pill for assessing H. Pylori infection, one for assessing pH and one for measuring ammonium ions as described above, and also one for measuring nitric oxide (NO). Nitric oxide is an important signaling molecule in the GI tract and plays an important role in intestinal inflammation and formation of ulcers. The combination of measuring pH, ammonium, and nitric oxide can not only determine the presence of H. pylori activity in the stomach (measured by ammonium sensor), but also assess the severity of the infection by measuring the presence of inflammation using the NO sensor. From the combined measurements an enhanced treatment strategy can be developed. The measurements are complementary and have a synergistic effect on the gut health assessment, where pH will help determine that the smart pill is in the stomach, ammonium ions will help determine that there is a presence of H. pylori activity, and nitric oxide will help determine that there is also presence of inflammation. This combined assessment of ammonium and nitric oxide for H. pylori is illustrated in Table 1 below.

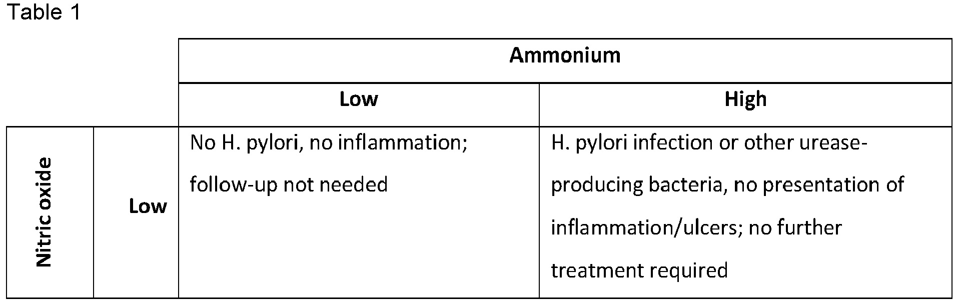

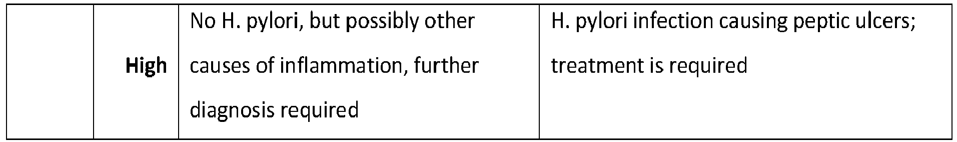

Low levels as indicated above means low elevation of the baseline values of ammonium ions, and a measured nitric oxide concentration not being elevated above a threshold. High levels means high/significant elevation of ammonium ions, which based on previous knowledge in the literature would mean that about 25% or more is a significant increase. For nitric oxide, a high level would mean an elevation above a threshold.

The present embodiment includes an amperometric readout for the monitoring of nitric oxide. It could be equipped with calibration and temperature compensation systems.

Accordingly, using the minimally invasive and sensitive methods of the invention will enable to determine the presence of H. pylori / H. pylori activity directly in the stomach, at the site of the infection, with a minimal patient burden. Detection of H. pylori activity in the stomach may be followed up by interventions and medications to treat the H. pylori infection. A figure depicting a smart pill comprising two sensors, pH sensor and ammonium sensor, is depicted in figure 3A, and a smart pill comprising three sensors, pH sensor, ammonium sensor and nitric oxide, is depicted in figure 3B. Other combinations of sensors are also possible, as described below.

In another aspect, a smart pill comprising at least two sensors may be used for assessing inflammatory bowel disease (IBD), including Crohn's disease (CD) and Ulcerative colitis (UC). As discussed above, the location and extent of the inflammation can be difficult to assess, and clinical evaluation is now mainly relying on invasive endoscopic examinations, fecal analysis, or evaluation of symptoms. By instead using a smart pill for assessing inflammation in the GI-tract *in vivo* and in real time, it will be possible to assist IBD patients in assessing inflammation status, to distinguish between CD and UC, and to simultaneously finetune nutritional intake. In one embodiment, a smart pill comprising two sensors are used, one for assessing pH, and one for detecting nitric oxide, as described above for the H. pylori assessments. Nitric oxide sensors can measure the extent and location of inflammation. It will help monitor disease progression, the effect of nutrition, effect of medication, and could distinguish Crohn's Disease from Ulcerative Colitis. The continuous measurements of pH may indicate when the smart pill exits one part, region or phase of the GI-tract and enters a new region, as the pH will typically remain more stable within one region, but change upon entering a new region. The pH profiles are measured and compared to reference data. By correlating these changes and comparisons to reference data with the time that the pill has spent in the GI-tract, it may be determined where the smart pill is currently located. By further correlating this location to the presence of inflammation as measured by the nitic oxide sensor, it may be determined which parts of the GI-tract that have an ongoing inflammation. This may further be correlated to the type of inflammation, to distinguish Crohn's Disease from Ulcerative Colitis, as they are typically present in different parts of the GI-tract. The present embodiment includes an amperometric readout for the monitoring of nitric oxide. It could be equipped with calibration and temperature compensation systems. For the specific monitoring of nitric oxide along the GI tract, an amperometric readout is employed, utilizing a nitric oxide permeable membrane.

In another embodiment, a smart pill comprising at least two sensors may be used for assessing levels of protein fermentation in a subject, where the two sensors measures pH and ammonium ions. Increased levels of protein fermentation may be detrimental to gut health, and measuring pH and ammonium ion concentrations in comparison to a reference value, which may be set or based on previous general knowledge or previous measurements in a subject, may assess levels of protein fermentation and the location thereof present in the GI-tract of said subject. The ammonium sensor may assess levels of protein fermentation while using the pH measurement to track the smart pill's location in the GI tract, i.e. using the pH sensor to determine where said levels of protein fermentation are present. Different treatments or diets may then be evaluated based on their effect on the protein fermentation levels.

In another embodiment, a smart pill comprising three sensors are used, one for assessing pH, one for detecting nitric oxide, and one for detecting ammonium ions, as described above. Detection of NO assesses the presence of inflammation, while ammonium also indicates excessive protein fermentation, which will thus make it possible to both assess inflammation status and simultaneous effects of nutritional intake. Ammonium ion concentrations may be in comparison to a reference value as indicated above, where a "low" value as indicated below refers to a low or no increase of said concentration. "Low" values of nitric oxide means that the measured concentrations are not above a threshold.

This combined assessment of ammonium and nitric oxide for IBD is illustrated in Table 2 below.

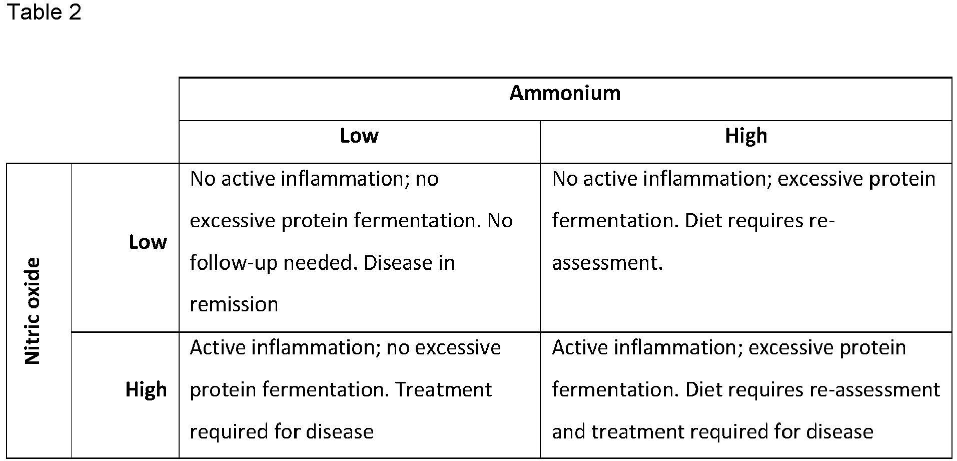

The smart pills of the present disclosure may also have further uses when it comes to gut health. Ammonium and nitric oxide both play an important role in the human nitrogen cycle. Disturbances of this cycle often originate in the gut. Examples are urea cycle enzyme deficiencies (dysregulation of conversion of ammonia nitrogen in urea), hyperammonemia (excess ammonia in the blood), hepatic encephalopathy (brain damage caused by liver damage and excess of toxins the blood such as ammonia). Ammonium measurements are thus also interesting in these conditions. Nitric oxide can be relevant in any use case where inflammation is expected in the gastrointestinal tract. It can also help distinguishing inflammatory disorders/diseases, from non-inflammatory conditions (such as irritable bowel syndrome). Measuring of systemic low-grade inflammation in the GI tract could be very powerful. Measuring both ammonium and nitric oxide in the gut can give more insights in these conditions, and also help with treatments or diets, as an important source of nitrogen is from our food intake.

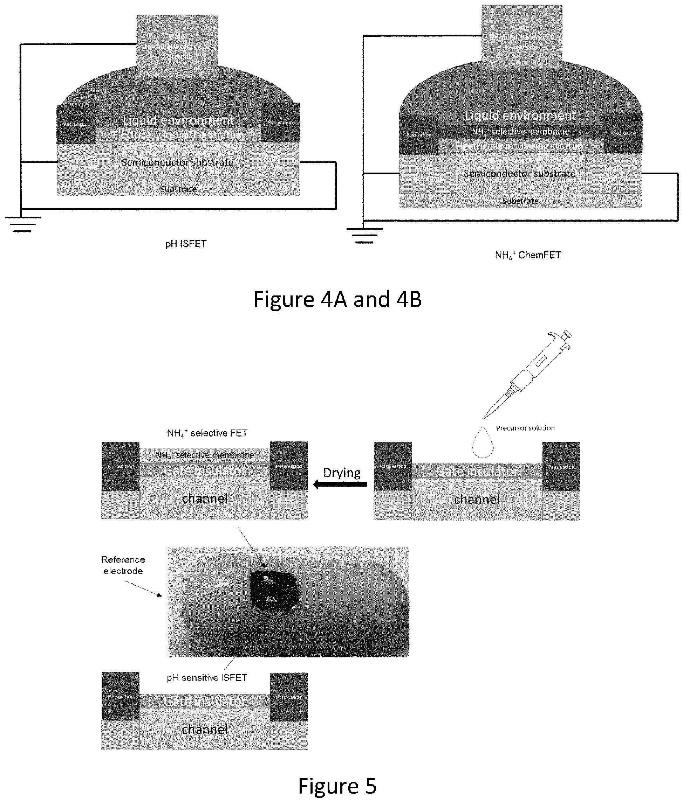

The smart pills of the present disclosure comprise at least two sensors and a reference electrode. The smart pills will also comprise a communication interface for live transmitting the measurement results of the sensors to a device outside of the body. One of the sensors will be a pH sensor for sensing the pH. This enables the location of the smart pill in the GI-tract. Measuring of pH may for example be performed with an ion selective field effect transistor (ISFET) and reference electrode. Thus, in one aspect the ingestible sensor includes at least one ISFET for the monitoring of pH. The ISFET comprises a body terminal, a source terminal, a drain terminal, an electrically insulating stratum and a gate terminal, as depicted in Figure 4A.

The body terminal is formed by a semiconductor substrate having a doping polarity. The electrically insulating stratum overlies at least the surface of the semiconductor substrate lying between the source and the drain. The gate terminal is a reference electrode, such as a silver/silver chloride reference electrode. The semiconductor is electrically insulated from the outer environment from the electrically insulated stratum so the potential at the gate terminal influences the current flow between the source terminal and the drain terminal.

The pill may also comprise an ammonium sensor. Ammonium ion levels are measured for example with a potentiometric technique based on ion selective field effect transistors (ISFET) or ion selective electrode (ISE). In some aspects, the ammonium sensor could be in the form of an ISE or an ammonium selective field effect transistor (ChemFET). In case an ISE is employed, the sensor comprises a metal electrode (like gold or platinum) coated by a solid contact made of a carbon component; this structure is hereby defined as electrode solid contact. In the present ISE sensor embodiment, the electrode solid contact can also be a screen-printed carbon electrode. The ISE embodiment includes a membrane selective to ammonium deposited on top of the electrode solid contact. The sensor includes a reference electrode like silver/silver chloride embedded in the ingestible capsule. The potentiometric response of the sensor is proportional to the concentration of ammonium ion present in the solution. The potential is measured over time, so the concentration of ammonium ion is monitored versus time. If the ChemFET is employed, the device of the present embodiment has the same body structure as an ISFET and an additional polymeric membrane (ammonium-selective) lying on top of the electrically insulating stratum, as depicted in Figure 4B. Sensor selectivity is guaranteed by a permeable polymeric membrane which has high capturing affinity to the target ion, and which is non-degradable. The membrane formulation typically includes a polymer (such as polyvinylchloride, PVC), a plasticizer, a selective ionophore for facilitating the transport of the ions across the membrane, and a lipophilic agent.

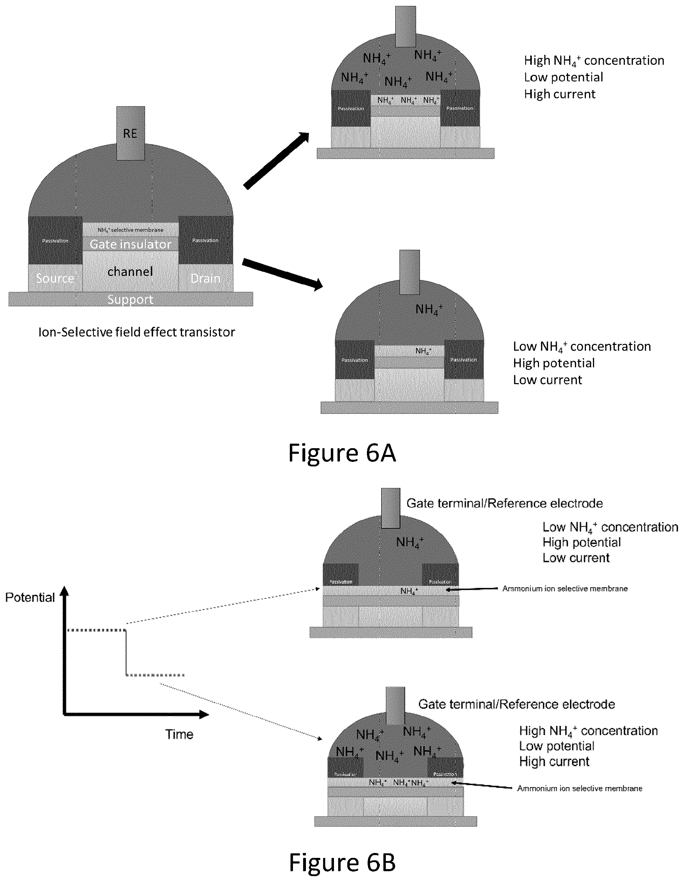

An illustration of an ingestible sensor (smart pill) comprising a pH sensor and an ammonium sensor showing the membrane deposition is depicted in Figure 5. The ammonium sensor has a semiconducting channel comprising a source (S) and a drain (D) metal contacts. The semiconductor channel is covered by a gate insulator or electrically insulating stratum. A precursor solution is applied to the gate insulator, dried, and an ammonium selective membrane is placed on top (the resulting film acts as ammonium selective membrane). The pH sensor is similar, but without the selective polymeric membrane. An example of a working mechanism for the ammonium sensor using an ISFET is showed in Figure 6A. The sensor comprises a source electrode, a drain electrode, a semiconducting channel, a reference electrode (RE), a gate insulator (electrically insulating stratum) and an ammonium selective membrane. Depending on the concentration of ammonium ions in the surrounding liquids, different potentials and currents will be attained, resulting in a low gate potential and high source/drain current if the ammonium concentration is high, and a high gate potential and low source/drain current if the concentration is low. This relationship of concentration to measured potential is illustrated in Figure 6B.

In an embodiment of using ISFET for the pH sensor and ChemFET for the ammonium sensor, in the ISFET, the gate insulator or electrically insulating stratum is sensitive to the pH of the liquid environment. In the ChemFET, the polymeric ammonium sensitive membrane lying on top of the electrically insulating stratum is the sensitive layer. The passivation of the electrically insulating stratum with a polymeric, ammonium-sensitive membrane makes the sensor sensitive to ammonium and insensitive to pH. The membrane comprises a polymer (like polyvinyl chloride, silicone or polyurethane) and a macrocyclic molecule that binds selectively to ammonium. This membrane has higher selectivity for ammonium compared to other competing ions, like sodium or potassium. The membrane is non-degradable, and it can be deposited onto the gate insulator from a precursor solution containing all the components. The ISFET and the ChemFET are embedded in an ingestible device to form a smart pill. The electrically insulating stratum of the ISFET is exposed to the outer environment. Similarly, the ammonium-selective membrane of the ChemFET is exposed. The rest of the ingestible device is encapsulated in an insulating shell. In this embodiment, the transmitter is located inside the body of the ingestible device.

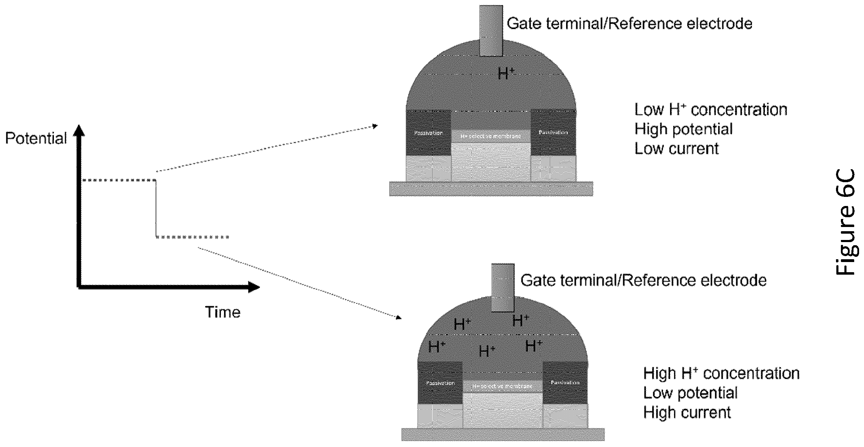

The ISFET operates at constant potential/constant current mode. The ISFET operates with a constant potential between the drain terminal (electrode) and the source terminal. The current flowing in the semiconductor channel is constant and its magnitude is determined by the second potential between the source terminal and gate terminal. In the case of the ISFET pH sensor, variations in pH (hydrogen ion concentration) provoke changes in the current flowing in the semiconductor substrate located between the source terminal and the drain terminal. Therefore, the potential between the gate terminal and the source terminal varies according to the concentration of analyte. The internal feedback system of the readout changes the potential between the source terminal and the gate terminal accordingly. This potential difference is the measured electronic signal as illustrated in Figure 6C. It can be converted to analyte concentration by applying the conversion determined during calibration.

The ChemFET operates at constant potential/constant current mode. The ChemFET operates with a constant potential between the drain terminal and the source terminal. The current flowing in the semiconductor structure is constant and its magnitude is determined by the second potential between the source terminal and gate terminal. Variations of the ammonium concentration in the ammonium-selective membrane provoke changes in the current flowing in the semiconductor substrate located between the source terminal and the drain terminal. Therefore, the potential between the gate terminal and the source terminal varies according to the concentration of ammonium. The feedback system of the readout changes the potential between the source terminal and the gate terminal accordingly. This potential difference is the measured electronic signal, as illustrated in figure 6B. It can be converted to analyte concentration by applying the conversion determined during calibration.

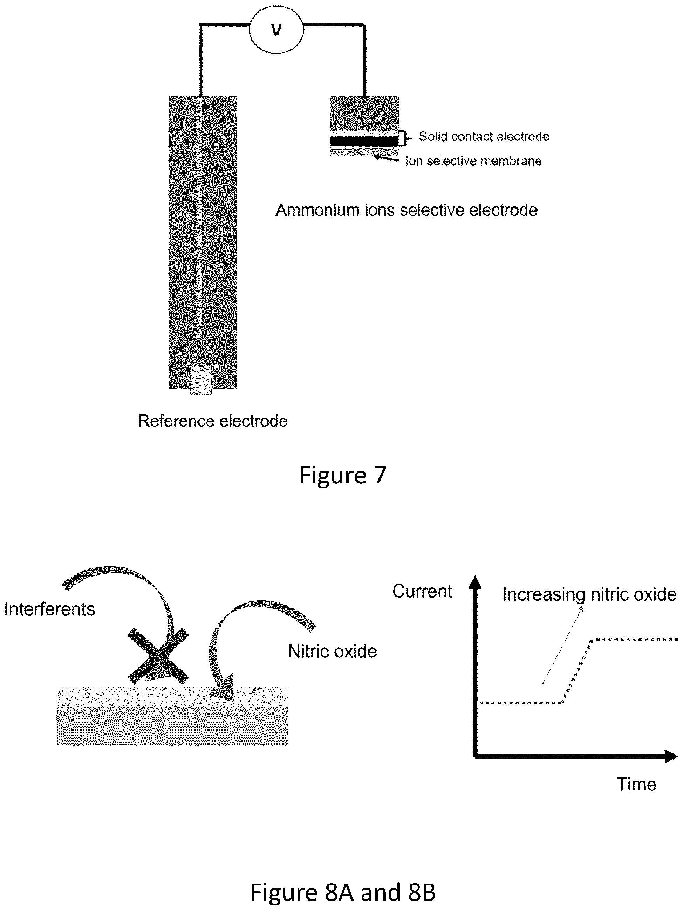

In the present ingestible device, the sensor for ammonium ions can also be an ion selective electrode (ISE), as illustrated in Figure 7. The sensor response can be monitored with a potentiometric readout. The potential response between the two electrodes is proportional to the concentration of ammonium ion present in the solution. The potential is measured during time, so the concentration of ammonium ion is monitored versus time, and measured in potentiometric configuration being an ISE.

The sensors of the smart pill may be calibrated before use. The ingestible sensor may be immersed in a minimum of two standard pH buffers to calibrate the pH ISFET. The buffers must reflect the pH values of the environment under investigation. In one calibration example, the ingestible sensor is immersed for one minute in each solution and the corresponding potential is recorded. The average potential value of the final 30 seconds at each pH solution is calculated. A calibration curve of the average potential versus pH is constructed for these measurements. For the ammonium sensor using ChemFET, the ingestible sensor may be immersed in a minimum of two standard ammonium solutions with a fixed ammonium concentration in order to calibrate the ChemFET. The ammonium standard solutions used during the calibration must reflect the ammonium concentration range of the environment under investigation. The ingestible sensor may be immersed for three minutes in each solution and the corresponding potential recorded. The average potential value of the final minute at each ammonium solution is calculated. A calibration curve of the average potential versus ammonium concentration is constructed for these measurements. Ammonium-selective membranes can be subject to interference by other compounds, such as potassium and sodium ions. In the present embodiment the effect of the interfering compounds could be compensated by considering the selectivity coefficient of the ammonium ion sensor towards potassium and sodium. For the ammonium sensor using ISE, the ingestible sensor may be immersed in a minimum of two standard ammonium solutions with a fixed ammonium concentration to calibrate the ISE. The ammonium standard solutions used during the calibration must reflect the ammonium concentration range of the environment under investigation. The ingestible sensor may be immersed for three minutes in each solution and the corresponding potential recorded. The average potential value of the final minute at each ammonium solution is calculated. A calibration curve of the average potential versus ammonium concentration is constructed for these measurements. Similar to the ammonium-selective membranes in the ChemFET, ammonium-selective membranes employed in the ISE can be subject to interference by potassium and sodium ions. In the present embodiment the effect of the interferents could compensated by considering the selectivity coefficient of the ammonium ion sensor towards potassium and sodium, which has been described in the art, see Fakih, I., Durnan, O., Mahvash, F. et al. Selective ion sensing with high resolution large area graphene field effect transistor arrays. Nat Commun 11, 3226 (2020).

Ammonium sensors for H. pylori has previously been described in the art. For example, WO201637663 discloses a H. pylori sensor including at least one chemical field-effect transistor. Compared to the present invention, this sensor is not part of a smart ingestible pill, and thus lacks the advantages thereof. In addition, the membrane used is degradable, compared to the membrane of the ammonium sensor of the present invention. That the membrane used is both highly selective and non-degradable has several advantages. Besides from being specific and sensitive, a non-degradable membrane allows for continuous measurements, as compared to a membrane that is degraded, and thus only may be used one time. The procedure and measurements cannot be repeated using a degradable membrane, as is possible using the membranes of the present invention, and further they cannot measure a decrease in the amount of ammonium present, only an initial increase may be assessed. As the membrane cannot be repaired, the sensors using this type of membrane allows only a one time measurement. Further, the membranes that measures degradation only measures indirect presence of ammonium ions, as it is assumed that it is the ammonium which degrades the membranes, but may be caused by other factors and thus result in a false positive test. In comparison, the membranes of the present invention are highly selective, they only bind to ammonium, and may thus directly measure the presence of ammonium ions.

The pill may also comprise a nitric oxide sensor. Nitric oxide may be measured with an amperometric technique in the range of potential 0.6-0.9 V. A conductive electrode is covered with a polymeric membrane which is permeable to nitric oxide but excludes interferents. The membrane is based on a fluoropolymer copolymer like Nafion. The electrode material can include gold, platinum, or carbon-based conductive electrode. Calibration and temperature compensation systems may be used, and for monitoring of nitric oxide along the GI tract, an amperometric readout is employed, utilizing a nitric oxide permeable electrode.

Thus, a metal electrode coated with a nitric oxide permeable membrane may be used as nitric oxide sensor. The electrode may be coated with a polymeric membrane which is selectively permeable to nitric oxide, and it allows nitric oxide to diffuse to the electrode surface and give the current signal. This membrane captures cationic electroactive species present in the GI tract and allows nitric oxide to pass through and reach the electrode surface. This membrane can be a cation permeable membrane like Nafion, PANAM, etc., and it is capable of excluding main interferents of nitric oxide present in the GI tract, as illustrated in Figure 8A. As discussed above, the nitric oxide is measured via an amperometric readout. The nitric oxide oxidation at the electrode surface is monitored continuously and there is a linear relationship between the current measured from the sensor and the concentration of nitric oxide. The current is measured along the GI tract and this value is converted to nitric oxide concentration by applying the calibration curve (calibration compensation). The read-out from the sensor is shown in Figure 8B, where an increased current is correlated with an increased nitric oxide presence in the environment of the sensor.

The nitric oxide sensor may also be calibrated. The nitric oxide sensor may be calibrated prior to the ingestion, with different standard solutions of nitric oxide. The calibration curve may be constructed by testing the ingestible device embedding the nitric oxide sensor in four different concentrations of nitric oxide relevant for the GI tract (0.5, 1, 10 and 100 µM). The calibration standards are created by dissolving commercial nitric oxide-releasing agents (e.g. - NONOate) in buffer solutions. The current of the sensor is recorded for each standard solution. The maximum current (baseline-subtracted) at each nitric oxide concentration is plotted versus the nitric oxide concentration to generate the calibration curve.

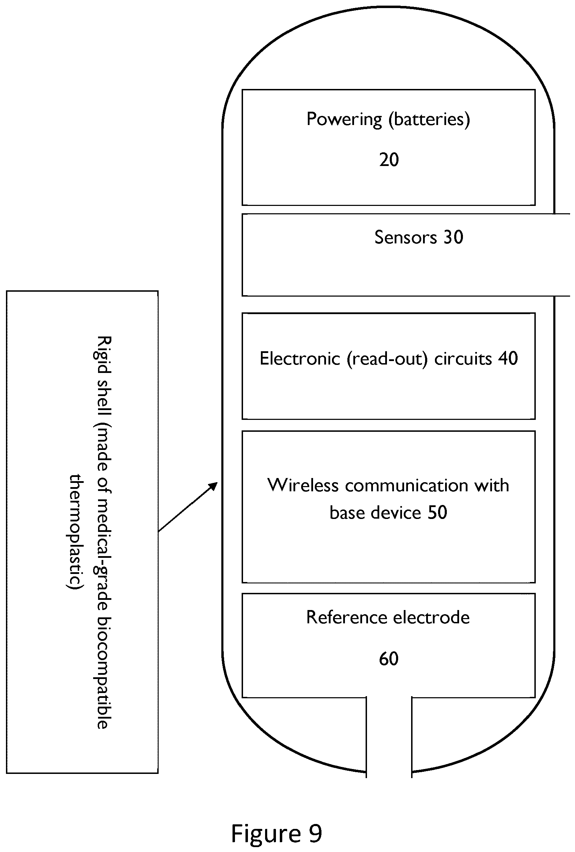

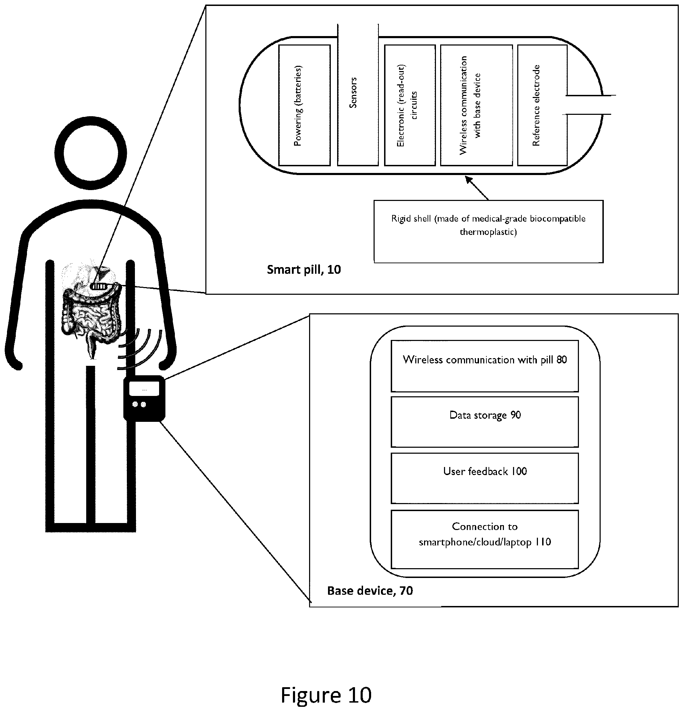

Accordingly, the present invention relates to an ingestible device, a smart pill, equipped with at least two sensors, a pH sensor, an ammonium ion sensor, and a sensor for nitric oxide (or a sub-selection thereof). The device can be ingested, and can continuously monitor the analytes along the gastrointestinal tract (GI tract). A schematic drawing of the different components of the smart pill system is illustrated in Figure 9. Only the sensors and the reference electrode are exposed to the GI fluids, as illustrated by the lack of borders towards the surroundings. The other components are shielded from any interaction with the outside environment, i.e. when digested the liquids and surroundings of the GI-tract. The ingestible sensor/smart pill 10 has batteries 20 for powering, an electronic read-out circuit (processing circuitry) 40 connecting the sensors 30 (including pH sensor 30a, and ammonium sensor 30b, and/or nitric oxide sensor 30c) and other parts of the system, a wireless communication component 50, comprising a communication interface, said interface comprising a transceiver, to connect (send/receive data) to a base device 70 (not shown), and a reference electrode 60 used as reference for all sensors. An illustration of the system comprising the smart pill 10 inside the body of a patient, and the base device 70 may be seen in Figure 10. In one embodiment, the electronic read-out circuit (processing circuitry) 40 may comprise a microcontroller configured to control the operation of smart pill 10. The microcontroller may include an algorithm or software program that aids in controlling the function or operation of the smart pill. The base device may comprise a wireless communication with the pill 80, such as a first communication interface comprising a transceiver for e.g. receiving measurement data from the sensors of the pill, a data storage/memory component 90, a connection to the user for user feedback 100, such as second communication interface, a connection to a server, terminal or device 110, a processing device 75, such as a smartphone, computer or a cloud, which may be a third communication interface. The second communication interface may be direct communication with the user from the base device, e.g. using audio-visual communications, such as a lamp (LED) that shows that the pill is still in the body (temperature of the pill has not dropped below body temp of 37 degrees). Thus, a temperature sensor may also be included. The ingestible sensor/smart pill transmits data wirelessly to a base device outside the body. The base device can be worn on the belt of the user, and may also be battery powered. It is able to store the received data from the ingestible device, and may provide user feedback. The base device can be connected to a server, terminal or other device, such as a computer or wireless device, e.g. laptop or smartphone, or may directly transmit the data to the cloud for data storage and further data analysis.

As discussed above, the methods and smart pills of the invention has several advantages over the prior art. No endoscopic procedure required, the sensors are integrated in a smart capsule ensuring a minimal burden to the patient. For the H. pylori application, the methods and pills are sensitive compared to urea breath tests, where there can be contamination from bacteria in the mouth, and stool samples. A profile of urease activity (and therefore H. pylori activity) can be constructed over time. The sensing membranes used are not soluble and can sense the target ion in the harsh conditions of the stomach environment. The smart pills are relatively inexpensive, as they do not require additional expensive equipment, compared to e.g. urea breath test requires isotope ratio mass spectrometer. Further, no isotope-labelled material is needed, as used for the 14C-urea breath tests. The methods allow for measurements directly in the stomach, and may thus follow changes in the measured analytes in real time *in vivo.* As no extra expensive equipment is needed (like for the urea breath tests), this is a very accessible solution of real time monitoring and very fast results, which can be performed at the general practitioner.

### EXAMPLE OPERATIONS

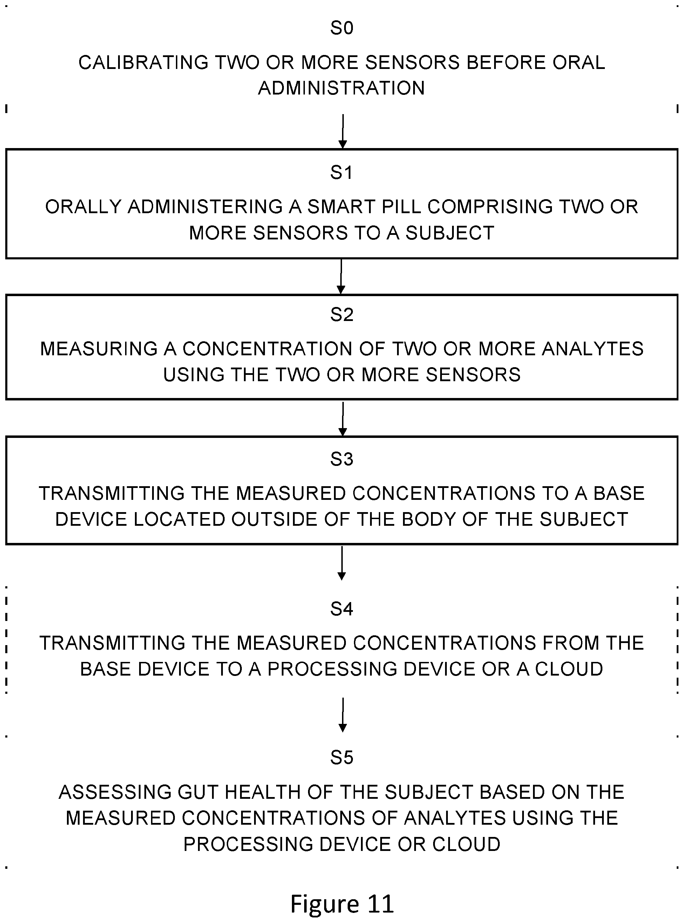

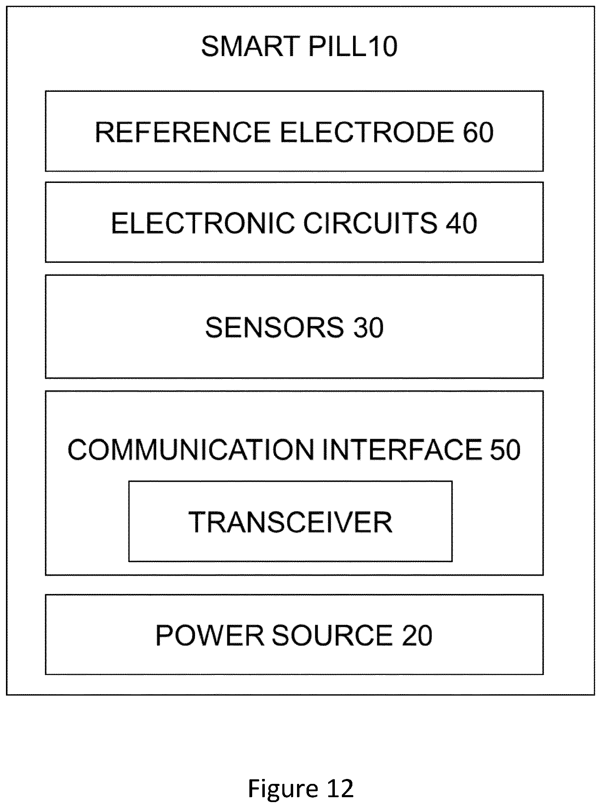

The proposed methods and devices will now be described in more detail referring to Figures 11 and 12. It should be appreciated that Figure 11 comprise some operations and modules which are illustrated with a solid border and some operations and modules which are illustrated with a dashed border. The operations and modules which are illustrated with solid border are operations which are comprised in the broadest example embodiment. The operations and modules which are illustrated with dashed border are example embodiments which may be comprised in, or a part of, or are further embodiments which may be taken in addition to the operations and modules of the broader example embodiments. It should be appreciated that the operations do not need to be performed in order. Furthermore, it should be appreciated that not all of the operations need to be performed.

Figure 11 illustrates a method for measuring analytes, i.e. measuring concentrations of analytes by measuring a potential induced by the presence of the analytes, which may be correlated to a concentration, in the gastrointestinal tract of a subject, the method comprising orally administering (S1) a smart pill to the subject, the smart pill comprising two or more sensors, a reference electrode, a power source, a communication interface and electronic circuits, each sensor being able to measure an analyte/concentrations of an analyte in the gastrointestinal tract of the subject, wherein the two or more sensors measure different distinct analytes; measuring (S2) a concentration of two or more analytes using the two or more sensors, wherein one of the sensors is a pH sensor for measuring hydrogen ions/hydrogen ion concentrations, and wherein the pH sensor is able to locate the smart pill in the gastrointestinal tract by correlating the measured hydrogen ion concentrations to a location in the gastrointestinal tract; and transmitting (S3), using the communication interface, the measured concentrations from the two or more sensors to a base device located outside of the body of the subject.

Before use, the method optionally comprises preparing the smart pill before use, including calibrating the two or more sensors of the smart pill before oral administration, for example using the calibration methods described above. The sensors will then be activated before use, which could be part of (intrinsic to) the step of orally administering the smart pill (S1).

The methods may further comprise transmitting (S4) data, including the measured concentrations of the two or more sensors, from the base device to a processing device or a cloud; and assessing (S5), gut health of the subject based on the measured concentrations of analytes of the two or more sensors using the processing device or cloud. The transmission of the data between the base device and the processing device may be wired or wireless. The data thus relates to the measured potential or correlated concentrations thereof.

In one aspect, at least one of the two or more sensors of the smart pill comprise a non-degradable permeable polymeric membrane, said membrane enabling continuous and/or repeated measurements using said sensors as described above. The pH sensor typically does not need such a membrane, while the ammonium ion sensor typically comprises one, and the nitric oxide sensor may also typically comprise such a membrane.

In one aspect not depicted in Figure 11, a subject is suffering from, or is suspected to be suffering from, a Helicobacter pylori infection and the smart pill comprises a pH sensor for measuring hydrogen ions/concentrations thereof and an ammonium sensor for measuring ammonium ions/concentrations thereof, wherein the measuring (S2) the two analytes/concentrations thereof and transmitting (S3) the measured concentrations comprises: continuously measuring (S21), using the pH sensor, hydrogen ions/concentrations thereof to determine a location of the smart pill in the gastrointestinal tract; determining (S22), based on the measured hydrogen ion concentrations, that the smart pill has reached the stomach; measuring (S23A), using the ammonium sensor, a baseline ammonium ion concentration; orally administering (S24) a predetermined amount of urea to the subject; continuously measuring (S25A), using the ammonium sensor, ammonium ion concentrations after administration of urea; and continuously transmitting (S31A), using the communication interface, the measured concentrations from the two sensors to the base device. The transmitted "concentrations" may be in the form of raw data (measured potential) and may be correlated to actual concentrations after being transmitted.

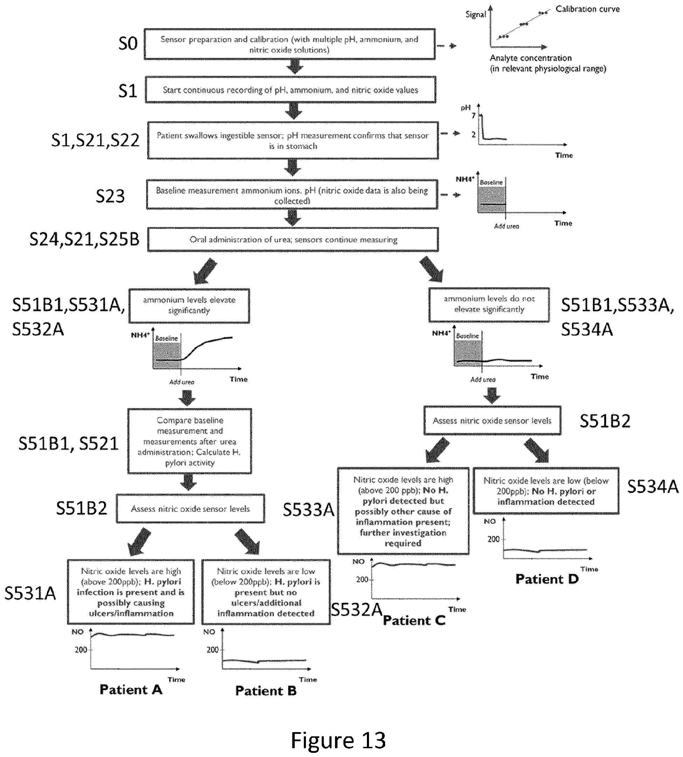

Gut health may be assessed based on the measured analytes/concentrations thereof by determining (S51A) a degree of elevation of ammonium ion concentrations in the gastrointestinal tract by subtracting the baseline ammonium ion concentration from the measured ammonium ion concentration(s) after administration of urea; correlating (S52) the degree of elevation of ammonium ion concentrations to the presence or absence of Helicobacter pylori activity in the stomach of the subject; and assessing (S53) gut health based on the presence or absence of Helicobacter pylori activity, wherein a significant degree of elevation is correlated with the presence of Helicobacter pylori activity and wherein a non-significant degree of elevation or lack of elevation is correlated with the absence of Helicobacter pylori activity. Thus, an elevation of the ammonium ion levels (concentrations) after administration of urea is measured, which may be correlated to H. pylori activity. The amount of elevation is dependent on the amount of urea used, hence the elevation must be related to said amount, and thus also the determination of if the elevation observed is significant or not of H. pylori activity and infection. In one example, as illustrated in the figures and example section below, the ammonium ion levels show a two, three or four fold increase (significant increase) when H. pylori activity is present, while being fairly stable when no H. pylori activity is present (see e.g. Figure 13). An increase of about 25% or more is labelled as significant, while no increase or an increase of less than 25% is labeled as a non-significant degree of elevation.

In another aspect not depicted in Figure 11, a subject is suffering from, or is suspected to be suffering from, a Helicobacter pylori infection and the smart pill comprises a pH sensor for measuring hydrogen ions, an ammonium sensor for measuring ammonium ions and a nitric oxide sensor for measuring nitric oxide, wherein the measuring (S2) the three analytes/concentrations thereof and transmitting (S3) the measured concentrations comprises: continuously measuring (S21), using the pH sensor, hydrogen ions/concentrations thereof to determine a location of the smart pill in the gastrointestinal tract; determining (S22), based on the measured hydrogen ion concentration, that the smart pill has reached the stomach; measuring (S23B), using the ammonium sensor, a baseline ammonium ion concentration; orally administering (S24) a predetermined amount of urea to the subject; continuously measuring (S25B), using the ammonium sensor and nitric oxide sensor, respectively, ammonium ion concentration and nitric oxide concentration after administration of urea; and continuously transmitting (S31B), using the communication interface, the measured concentrations from the three sensors to the base device.

Gut health may be assessed based on the measured analyte concentrations by determining (S51B1) a degree of elevation of ammonium ion concentrations in the gastrointestinal tract by subtracting the baseline ammonium ion concentration from the measured ammonium ion concentration after administration of urea and determining (S51B2) a degree of elevation of nitric oxide concentrations in the gastrointestinal tract by comparing the measured nitric oxide concentrations to a predetermined threshold; correlating (S521) the degree of elevation of ammonium ion concentrations to the presence or absence of Helicobacter pylori activity in the stomach of the subject; correlating (S522) the degree of elevation of nitric oxide concentrations to the presence or absence of ulcers and/or inflammation, wherein a measured concentration of nitric oxide being above or equal to the predetermined threshold is considered as a significant degree of elevation; and assessing (S53B) gut health based on the presence or absence of Helicobacter pylori activity and ulcers and/or inflammation, wherein (S531A) a significant degree of elevation of ammonium ion concentrations and nitric oxide concentrations are correlated with the presence of Helicobacter pylori activity and ulcers and/or inflammation, wherein (S532A) a significant degree of elevation of ammonium ion concentrations and a non-significant degree of elevation of nitric oxide concentrations are correlated with the presence of Helicobacter pylori activity but no ulcers and/or inflammation, wherein (S533A) a non-significant degree of elevation or no elevation of ammonium ion concentrations and a significant degree of elevation of nitric oxide concentrations are correlated with the absence of Helicobacter pylori activity and the presence of ulcers and/or inflammation, and wherein (S534A) a non-significant degree of elevation or no elevation of ammonium ion concentrations and a non-significant degree of elevation of nitric oxide concentrations are correlated with the absence of Helicobacter pylori activity and ulcers and/or inflammation. The ammonium ion levels compared to a baseline measurement is determined as a significant elevation as described above, while the measured nitric oxide concentrations are compared to a threshold. As described in the example section below, the nitric oxide threshold may be 200 ppb.

In another aspect not depicted in Figure 11, a subject is suffering from, or is suspected to be suffering from, inflammatory bowel disease and/or suspected of presenting increased protein fermentation, and the smart pill comprises a pH sensor for measuring hydrogen ions/concentrations thereof and a nitric oxide sensor for measuring nitric oxide/concentrations thereof or an ammonium sensor for measuring ammonium ions/concentrations thereof, wherein the measuring (S2) the two analytes and transmitting (S3) the measured concentrations comprises: continuously measuring (S210), using the pH sensor, hydrogen ions/concentrations thereof to determine, based on the hydrogen ion concentration and a time that has passed from oral administration of the smart pill, a location of the smart pill in the gastrointestinal tract; continuously measuring (S220A), using the nitric oxide sensor or ammonium sensor, nitric oxide concentrations or ammonium ion concentrations as the smart pill travels through the gastrointestinal tract, wherein said measuring includes measuring in the stomach (S2201), measuring in the small intestine (S2202) and measuring in the large intestine (S2203); continuously transmitting (S310A), using the communication interface, the measured concentrations from the two sensors to the base device.

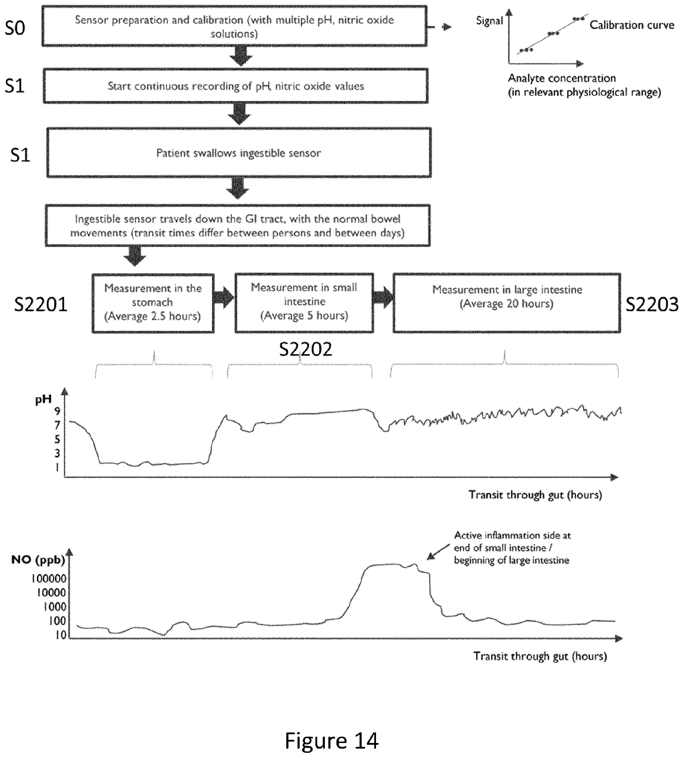

Gut health may be assessed based on the measured analytes/concentrations thereof, wherein the smart pill comprises a nitric oxide sensor, by determining (S510A1) a degree of elevation of nitric oxide concentrations in the gastrointestinal tract by comparing the measured nitric oxide concentrations to a predetermined threshold; correlating (S5210A) the degree of elevation of nitric oxide concentrations to the presence or absence of ulcers and/or inflammation, wherein a measured concentration of nitric oxide being above or equal to the predetermined threshold is considered as a significant degree of elevation; and assessing (S530A) gut health based on the presence or absence of ulcers and/or inflammation, wherein a significant degree of elevation nitric oxide concentrations are correlated with the presence of ulcers and/or inflammation, and wherein a non-significant degree of elevation of nitric oxide concentrations are correlated with the absence of ulcers and inflammation. The method further provides for distinguishing between UC and CD by determining (S510A1) a degree of elevation of nitric oxide concentrations in the gastrointestinal tract by comparing the measured nitric oxide concentrations to a predetermined threshold is performed at different locations in the gastrointestinal tract, including the stomach, the small intestine and the large intestine, and wherein a significant degree of elevation of nitric oxide concentrations restricted to the large intestine is indicative of Ulcerative Colitis, a significant degree of elevation of nitric oxide concentrations in other parts of the gastrointestinal tract is indicative of Crohn's Disease. As described in the example section below, the nitric oxide threshold may be 200 ppb. As shown in the example section below, measurements in the stomach may on average last about 2-3 hours, measurements in the small intestine will last about 5 hours and in the large intestines about 20 hours, and depending on the time from the start of measurements, and the pH profile, it is possible to determine in which part the smart pill is currently located, as illustrated in Figure 14. By correlating the nitric oxide measurements to the current location obtained by the hydrogen ions measurements, it is possible to determine the site of inflammation. In one example of assessing 215 healthy volunteers, the following average pH was measured Stomach: mean 1.9 (range: 0.7-4.6), Small intestine: mean: 7.2 (range: 6.3-7.8), Large intestine: mean: 7.0 (range: 5.7-8.1) (it is important to note that the values may change a bit in health affected patients). Accordingly, based on pH profiles and timing information, the current location of the pill may be determined.

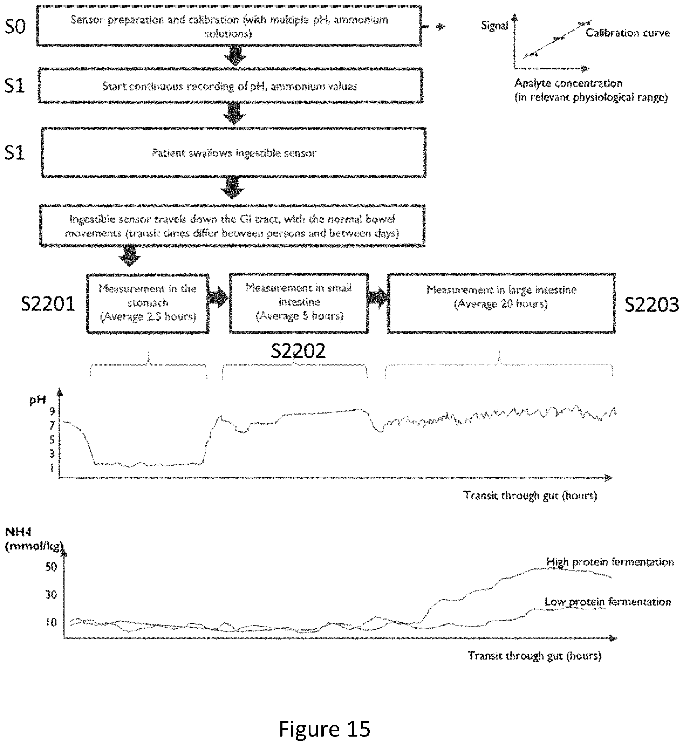

Alternatively, gut health may be assessed based on the measured analytes/concentrations thereof, wherein the smart pill comprises a ammonium sensor, by determining (S510A2) a degree of elevation of ammonium ion concentrations in the gastrointestinal tract by comparing the measured ammonium ion concentrations to a reference value; correlating (S5210B) the degree of elevation of ammonium ion concentrations to a level of protein fermentation; and assessing (S530A) gut health based on the level of protein fermentation, wherein a significant degree of elevation of ammonium ion concentrations indicates a high level of protein fermentation, a non-significant degree of elevation of ammonium ion concentrations indicates a low level of protein fermentation, and/or a positive degree of elevation of ammonium ion concentrations compared to the reference value indicates an increased level of protein fermentation and a negative degree of elevation of ammonium ion concentration compared to the reference value indicates a decreased level of protein fermentation. Thus, by comparing the measured ammonium levels compared to a reference value, it may be determined if the subject is suffering from a high level of protein fermentation or not, and it may also be determined if the ammonium levels, i.e. protein fermentation, is increasing or decreasing based on if the levels are increasing (positive degree of elevation) or decreasing (negative degree of elevation). As described above and in the example section below, this reference value may be determined from the literature or based on previous knowledge and/or measurements of the subject being analyzed, where different diets can for example be compared in the same subject to determine the level of protein fermentation. As shown in Figure 15, a level of 0-15 mmol/kg is labelled as a low level of protein fermentation being present, where a level of 50 mmol/kg is labelled as a high degree of high protein fermentation being present. Thus, the reference value may be used for a personalized assessment, where from 15-50 mmol/kg may be labelled differently depending on the subject assessed. Typically, if used as a threshold value, a level above 30 mmol/kg would be deemed a significant degree of elevation of the protein fermentation levels.

In another aspect not depicted in Figure 11, a subject is suffering from, or is suspected to be suffering from inflammatory bowel disease and the smart pill comprises a pH sensor for measuring hydrogen ions/concentrations thereof, an nitric oxide sensor for measuring nitric oxide/concentrations thereof and an ammonium sensor for measuring ammonium ions/concentrations thereof, wherein the measuring (S2) the three analytes/concentrations thereof and transmitting (S3) the measured concentrations comprises: continuously measuring (S210), using the pH sensor, hydrogen ions to determine, based on the hydrogen ion concentration and a time that has passed from oral administration of the smart pill, a location of the smart pill in the gastrointestinal tract; continuously measuring (S220B), using the nitric oxide sensor and ammonium sensor, respectively, nitric oxide concentrations and ammonium ion concentrations as the smart pill travels through the gastrointestinal tract, wherein said measuring includes measuring in the stomach (S2201), measuring in the small intestine (S2202) and measuring in the large intestine (S2203); continuously transmitting (S310B), using the communication interface, the measured concentrations from the three sensors to the base device.

Gut health may be assessed based on the measured analytes/concentrations thereof by determining (S510B) a degree of elevation of nitric oxide concentrations in the gastrointestinal tract by comparing the measured nitric oxide concentrations to a predetermined threshold and a degree of elevation of ammonium ion concentrations in the gastrointestinal tract by comparing the measured ammonium ion concentrations to a reference value; correlating (S5210) the degree of elevation of nitric oxide concentrations to the presence or absence of ulcers and/or inflammation; correlating (S5220) the degree of elevation of ammonium ion concentrations to a level of protein fermentation; and assessing (S530B) gut health based on the presence or absence of ulcers and/or inflammation and level of protein fermentation, wherein (S5301) a significant degree of elevation of nitric oxide concentrations and ammonium ion concentrations or a positive degree of elevation of ammonium ion concentrations are correlated with the presence of ulcers and/or inflammation and a high level of protein fermentation and/or an increased level of protein fermentation, wherein (S5302) a significant degree of elevation of nitric oxide concentrations and a non-significant or negative degree of elevation of ammonium ion concentrations are correlated with the presence of ulcers and/or inflammation and a low level of protein fermentation and/or decreased level of protein fermentation, wherein (S5303) a non-significant degree of elevation or no elevation of nitric oxide concentrations and a significant or positive degree of elevation of ammonium ion concentrations are correlated with the absence of ulcers and/or inflammation and a high level of protein fermentation and/or an increased level of protein fermentation, and wherein (S5304) a non-significant degree of elevation or no elevation of nitric oxide concentrations or ammonium ion concentrations and a negative degree of elevation of ammonium ion concentrations are correlated with the absence of ulcers, inflammation and a low level of protein fermentation and/or a decreased level of protein fermentation. As mentioned above, the threshold for nitric oxide t may be 200 ppb, and the reference value of ammonium ion concentrations may be between 15-50 mmol/kg, such as 20, 25, 30, 35, 40 or 45, where an ammonium ion level above said reference value may be correlated to a significant degree of elevation, i.e. increased protein fermentation, and a level below said reference value may be correlated to a non-significant degree of elevation. A decrease in elevation compared to the reference value, when the reference value is set based on previous measurements of the subject itself, may be a sign of a decrease of protein fermentation, and vice versa.

A smart pill of the invention will now be described referring to Figure 12. Figure 12 illustrates a block diagram of a smart pill of the invention, comprising a power source 20, typically some sort of batteries, for powering the pill, sensors 30 including the two or more sensors above, i.e. a pH sensor and one or more of an ammonium sensor and a nitric oxide sensor, and possibly also additional sensors for measuring background ions. The sensors being configured to measure analytes/concentrations of different analytes inside the gastrointestinal tract of a subject, at least one of the two or more sensors comprising a non-degradable membrane, e.g. an ammonium sensor or a nitric oxide sensor. The sensors are connected to a communication interface 50 using electronic circuits 40, the communication interface comprising a transceiver (transmitter/ receiver) for transmitting and receiving information between the sensors and a base device 70 (not shown). A reference electrode 60 is also included in the smart pill, used as reference for the sensors. The electronic circuits (40) enables a connection between the sensors (30), the reference electrode (60) and the communication interface (50), and being configured to convey data from the sensors (30) to the communication interface (50).

The content of this disclosure thus enables measurements of analytes in the gastrointestinal tract in vivo, assessing the concentrations thereof, which may be used for assessing gut health of a subject in need thereof. In the drawings and specification, there have been disclosed exemplary aspects of the disclosure. However, many variations and modifications can be made to these aspects without substantially departing from the principles of the present disclosure. Thus, the disclosure should be regarded as illustrative rather than restrictive, and not as being limited to the particular aspects discussed above. Accordingly, although specific terms are employed, they are used in a generic and descriptive sense only and not for purposes of limitation.

The description of the example embodiments provided herein have been presented for purposes of illustration. The description is not intended to be exhaustive or to limit example embodiments to the precise form disclosed, and modifications and variations are possible in light of the above teachings or may be acquired from practice of various alternatives to the provided embodiments. The examples discussed herein were chosen and described in order to explain the principles and the nature of various example embodiments and its practical application to enable one skilled in the art to utilize the example embodiments in various manners and with various modifications as are suited to the particular use contemplated. The features of the embodiments described herein may be combined in all possible combinations of methods, products, and systems. It should be appreciated that the example embodiments presented herein may be practiced in any combination with each other.

It should be noted that the word "comprising" does not necessarily exclude the presence of other elements or steps than those listed and the words "a" or "an" preceding an element do not exclude the presence of a plurality of such elements. It should further be noted that any reference signs do not limit the scope of the claims, that the example embodiments may be realized in the broadest sense of the claims.

### EXAMPLES

### Example 1, sensor administration and assessment of H. pylori activity

An overview of the present example may be seen in Figure 13. In the present example, four patients exhibiting mild to severe gastrointestinal discomfort are assessed for gut health:
Patient A: Presenting strong stomach pain, nausea, and possible ulcers
Patient B: Presenting mild stomach discomfort
Patient C: Presenting strong stomach pain, nausea, and possible ulcers
Patient D: Presenting mild stomach discomfort

For each patient, the smart pill comprising three sensors, pH, ammonium and nitric oxide, is first calibrated by measuring in pH, ammonium, and nitric oxide solutions of known concentrations. The sensors are then activated to begin continuous recording of pH, ammonium, and nitric oxide values. The patients then ingests the pill and the pH value is monitored to establish when the pill has reached the stomach. Once in the stomach a baseline ammonium measurement is established over several minutes. A known amount of urea is then administered to the patient.

At this step, the cause of the patients' symptoms, despite being similar, could be differentiated by the ammonium sensor data. If an H. pylori infection contributes to the patient's stomach pain, an increase in the ammonium sensor values will be observed (Patient A and Patient B). If the patient does not have an H. Pylori infection, their ammonium sensor data will not rise significantly above the baseline level (Patient C and Patient D).

Furthermore, the nitric oxide sensor data can be investigated here to elucidate additional causes of the inflammation. A nitric oxide sensor value above 200 ppb (as in Patient A's sensor data) is attributed to high inflammation levels stemming from peptic ulcers. With both high ammonium sensor data and high nitric oxide sensor data, it is probable that Patient A's positive H. Pylori infection is contributing to their inflammation/peptic ulcers.

The low nitric oxide sensor data (below 200 ppb) in Patient B indicates the patient's stomach discomfort is likely limited to the H. pylori infection (and no further inflammation is detected). Similarly, in the case of Patient C and patient D, the nitric oxide sensor data could provide distinguishing information on the inflammatory status in the patient's stomach. If no H. pylori infection and low nitric oxide sensor values (less than 200 ppb) are observed, the patient does not have significant inflammation in the stomach (Patient D). On the other hand, as is demonstrated in Patient C, nitric oxide sensor values above 200 ppb indicate that inflammation or ulcers are likely contributing to the patient's symptoms. But importantly, an H. pylori infection is not causing these ulcers/inflammation in Patient C.

With this ammonium and nitric oxide sensor information, clinicians can make a more informed decision on the individualized treatment of Patients A, B, C, & D.

### Example 2, sensor administration and assessment of nitric oxide in IBD

In the present example, illustrated in Figure 14, a patient with inflammatory bowel disease is investigated. The clinician is interested in the levels of inflammation in the bowel of this patient to monitor the disease progression and to look at the effects of treatment.

The sensors may be calibrated, and are then activated to begin continuous recording of pH and nitric oxide values. The patient swallows the ingestible sensors. The smart pill will travel down the normal route of the food and will on average stay 2.5 hours in stomach, 5 hours in the small intestine, and 20 hours in the large intestine. The pH profile, in combination with the transit times, can help to give a global location of the pill. Transitions between the different part of the GI tract can be observed.

The nitric oxide levels are indicative of inflammation. In this example a large increase of nitric oxide is observed at the end of the small intestine/beginning of large intestine. Most likely due to an active inflammation site.

### Example 3, sensor administration and assessment of protein fermentation via ammonium sensing

In the present example, as illustrated in Figure 15, we compare two different diets: one high in protein and low in fiber, and one low in protein and high in fiber, to observe differences in protein fermentation. Protein fermentation occurs most often in the distal part of the colon. Protein that did get absorbed by the body in the small intestine is being fermented in the large intestine by the gut microbiota. During this fermentation process ammonium is being produced, among other metabolites. The compounds produced during protein fermentation can have a negative impact on gut health and are being seen as one of the risk factors for inflammatory bowel diseases. Protein fermentation can be tuned by changing the diet (balance between intake of dietary fiber and protein). Especially in conditions where uptake of nutrients is disturbed (like in IBD), additional attention needs to be paid to the diet composition.

In the present example, the smart pill is first calibrated by measuring in multiple pH, and ammonium solutions of known concentrations. The sensors are then activated to begin continuous recording of pH and ammonium values. The patient swallows the ingestible sensors, and the smart pill will travel down the normal route of the food and will on average stay 2.5 hours in stomach, 5 hours in the small intestine, and 20 hours in the large intestine.

The pH profile, in combination with the transit times, can help to give a global location of the pill. Transitions between the different part of the GI tract can be observed.

The measured ammonium concentrations are indicative of protein fermentation levels. From this data, an individual nutrition plan can be prescribed to optimize protein levels and minimize intestinal discomfort.

## Claims

1. A method for measuring concentrations of analytes in a gastrointestinal tract of a subject, the method comprising:
orally administering (S1) a smart pill to the subject, the smart pill comprising two or more sensors, a reference electrode, a power source, a communication interface and electronic circuits, each sensor being able to measure a concentration of an analyte in the gastrointestinal tract of the subject, wherein the two or more sensors measure different analytes;
measuring (S2) a concentration of two or more analytes using the two or more sensors, wherein one of the sensors is a pH sensor for measuring hydrogen ion concentrations as a concentration of one analyte, and wherein the pH sensor is able to locate the smart pill in the gastrointestinal tract by correlating the measured hydrogen ion concentrations to a location in the gastrointestinal tract; and
transmitting (S3), using the communication interface, the measured concentrations from the two or more sensors to a base device located outside of a body of the subject.

2. The method according to claim 1, further comprising assessing gut health of the subject based on the measured analyte concentrations by:
transmitting (S4) the measured concentrations of analytes from the base device to a processing device or a cloud; and
assessing (S5), gut health of the subject based on the measured concentrations of analytes of the two or more sensors using the processing device or cloud.

3. The method according to claims 1-2, wherein at least one of the two or more sensors of the smart pill comprise a non-degradable permeable polymeric membrane, said membrane enabling continuous and/or repeated measurements using said sensors.

4. The method according to anyone of claims 2-3, wherein the subject is suspected to be suffering from a *Helicobacter pylori* infection and the smart pill comprises an ammonium sensor for measuring ammonium ion concentrations as a concentration of one analyte, wherein the measuring (S2) a concentration of the two analytes and transmitting (S3) the measured concentrations comprises:
continuously measuring (S21), using the pH sensor, hydrogen ion concentrations to determine a location of the smart pill in the gastrointestinal tract;
determining (S22), based on the measured hydrogen ion concentrations, that the smart pill has reached a stomach of the subject;
measuring (S23A), using the ammonium sensor, a baseline ammonium ion concentration;
orally administering (S24) a predetermined amount of urea to the subject;
continuously measuring (S25A), using the ammonium sensor, ammonium ion concentrations after administration of urea; and
continuously transmitting (S31A), using the communication interface, the measured concentrations from the two sensors to the base device.

5. The method according to claim 4, wherein assessing (S5) gut health of the subject based on the measured analyte concentrations comprises:
determining (S51A) a degree of elevation of ammonium ion concentrations in the gastrointestinal tract by subtracting the baseline ammonium ion concentration from the measured ammonium ion concentrations after administration of urea;
correlating (S52) the degree of elevation of ammonium ion concentrations to the presence or absence of *Helicobacter pylori* activity in the stomach of the subject; and
assessing (S53) gut health of the subject based on the presence or absence of *Helicobacter pylori* activity, wherein a significant degree of elevation is correlated with the presence of *Helicobacter pylori* activity and wherein a non-significant degree of elevation or lack of elevation is correlated with the absence of *Helicobacter pylori* activity.

6. The method according to anyone of claims 2-3, wherein the subject is suspected to be suffering from a *Helicobacter pylori* infection and the smart pill comprises an ammonium sensor for measuring ammonium ion concentrations as a concentration of one analyte and a nitric oxide sensor for measuring nitric oxide concentrations as a concentration of one analyte, wherein the measuring (S2) a concentration of the three analytes and transmitting (S3) the measured concentrations comprises:
continuously measuring (S21), using the pH sensor, hydrogen ion concentrations to determine a location of the smart pill in the gastrointestinal tract;
determining (S22), based on the measured hydrogen ion concentration, that the smart pill has reached a stomach of the subject;
measuring (S23B), using the ammonium sensor, a baseline ammonium ion concentration;
orally administering (S24) a predetermined amount of urea to the subject;
continuously measuring (S25B), using the ammonium sensor and nitric oxide sensor, respectively, ammonium ion concentrations and nitric oxide concentrations after administration of urea; and
continuously transmitting (S31B), using the communication interface, the measured concentrations from the three sensors to the base device.

7. The method according to claim 6, wherein assessing (S5) gut health of the subject based on the measured analyte concentrations comprises:
determining (S51B1) a degree of elevation of ammonium ion concentrations in the gastrointestinal tract by subtracting the baseline ammonium ion concentration from the measured ammonium ion concentrations after administration of urea and determining (S51B2) a degree of elevation of nitric oxide concentrations in the gastrointestinal tract by comparing the measured nitric oxide concentrations to a predetermined threshold;
correlating (S521) the degree of elevation of ammonium ion concentrations to the presence or absence of *Helicobacter pylori* activity in the stomach of the subject;
correlating (S522) the degree of elevation of nitric oxide concentrations to the presence or absence of ulcers and/or inflammation, wherein a measured concentration of nitric oxide being above or equal to the predetermined threshold is considered as a significant degree of elevation; and
assessing (S53B) gut health based on the presence or absence of *Helicobacter pylori* activity and ulcers and/or inflammation, wherein (S531A) a significant degree of elevation of ammonium ion concentrations and nitric oxide concentrations are correlated with the presence of *Helicobacter pylori* activity and ulcers and/or inflammation, wherein (S532A) a significant degree of elevation of ammonium ion concentrations and a non-significant degree of elevation of nitric oxide concentrations are correlated with the presence of *Helicobacter pylori* activity but no ulcers and/or inflammation, wherein (S533A) a non-significant degree of elevation or no elevation of ammonium ion concentrations and a significant degree of elevation of nitric oxide concentrations are correlated with the absence of *Helicobacter pylori* activity and the presence of ulcers and/or inflammation, and wherein (S534A) a non-significant degree of elevation or no elevation of ammonium ion concentrations and a non-significant degree of elevation of nitric oxide concentrations are correlated with the absence of *Helicobacter pylori* activity and ulcers and/or inflammation.

8. The method according to anyone of claims 2-3, wherein the subject is suffering from, or is suspected to be suffering from, inflammatory bowel disease and/or suspected of presenting increased protein fermentation, and the smart pill comprises a nitric oxide sensor for measuring nitric oxide concentrations or an ammonium sensor for measuring ammonium ion concentrations as a concentration of one analyte, wherein the measuring (S2) a concentration of the two analytes and transmitting (S3) the measured concentrations comprises:
continuously measuring (S210), using the pH sensor, hydrogen ion concentrations to determine, based on the hydrogen ion concentrations and a time that has passed from oral administration of the smart pill, a location of the smart pill in the gastrointestinal tract;
continuously measuring (S220A), using the nitric oxide sensor or ammonium sensor, nitric oxide concentrations or ammonium ion concentrations as the smart pill travels through the gastrointestinal tract, wherein said measuring includes measuring in stomach (S2201), measuring in small intestine (S2202) and measuring in large intestine (S2203);
continuously transmitting (S310A), using the communication interface, the measured concentrations from the two sensors to the base device.

9. The method according to claim 8, wherein the smart pill comprises a nitric oxide sensor and wherein assessing (S5) gut health of the subject based on the measured analyte concentrations comprises:
determining (S510A1) a degree of elevation of nitric oxide concentrations in the gastrointestinal tract by comparing the measured nitric oxide concentrations to a predetermined threshold;
correlating (S5210A) the degree of elevation of nitric oxide concentrations to the presence or absence of ulcers and/or inflammation, wherein a measured concentration of nitric oxide being above or equal to the predetermined threshold is considered as a significant degree of elevation; and
assessing (S530A) gut health based on the presence or absence of ulcers and/or inflammation, wherein a significant degree of elevation nitric oxide concentrations are correlated with the presence of ulcers and/or inflammation, and wherein a non-significant degree of elevation of nitric oxide concentrations are correlated with the absence of ulcers and inflammation.

10. The method according to claim 9, wherein determining (S510A1) a degree of elevation of nitric oxide concentrations in the gastrointestinal tract by comparing the measured nitric oxide concentrations to a predetermined threshold is performed at different locations in the gastrointestinal tract of the subject, including the stomach, the small intestine and the large intestine, and wherein a significant degree of elevation of nitric oxide concentrations restricted to the large intestine is indicative of Ulcerative Colitis, a significant degree of elevation of nitric oxide concentrations throughout the gastrointestinal tract, not restricted to the large intestine, is indicative of Crohn's Disease.

11. The method according to claim 9, wherein the smart pill comprises an ammonium ion sensor and wherein assessing (S5) gut health of the subject based on the measured analyte concentrations comprises:
determining (S510A2) a degree of elevation of ammonium ion concentrations in the gastrointestinal tract by comparing the measured ammonium ion concentrations to a reference value;
correlating (S5210B) the degree of elevation of ammonium ion concentrations to a level of protein fermentation; and
assessing (S530A) gut health based on the level of protein fermentation, wherein a significant degree of elevation of ammonium ion concentrations indicates a high level of protein fermentation, a non-significant degree of elevation of ammonium ion concentrations indicates a low level of protein fermentation, and/or a positive degree of elevation of ammonium ion concentrations compared to the reference value indicates an increased level of protein fermentation and a negative degree of elevation of ammonium ion concentrations compared to the reference value indicates a decreased level of protein fermentation.

12. The method according to claim any one of the preceding claims, further comprising:
calibrating (S0) the two or more sensors before oral administration to the subject.

13. A smart pill (10), configured to operate inside a gastrointestinal tract of a subject and being capable of measuring analyte concentrations therein, the smart pill comprising:
a power source (20),
two or more sensors (30), being configured to measure concentrations of different analytes inside the gastrointestinal tract of the subject, at least one of the two or more sensors comprising a non-degradable membrane,
a reference electrode (60),
a communication interface (50) comprising a transceiver for enabling wireless communication between the smart pill (10) and a base device (70) and being configured to transmit and receive data between the sensors (30) and the base device (70), and
electronic circuits (40) for enabling a connection between the sensors (30), the reference electrode (60) and the communication interface (50), and being configured to convey data from the sensors (30) to the communication interface (50).

14. The smart pill (10) according to claim 13, further comprising one or more additional sensor, wherein said additional sensor is able to correct for interference of other compounds.

15. The smart pill (10) according to claims 13-14, configured to perform the method of any one of claims 1-12.
